# FASCICULE DE BREVET EUROPEEN

(11) **EP 3 712 244 B1**
(45) Date de publication et mention de la délivrance du brevet: **01.05.2024**
(21) Numéro de dépôt: 20163255.1
(22) Date de dépôt: 16.03.2020
(51) Int. Cl.: C12M 1/32, C12M 3/06

(54) **PROCÉDÉ D'ENRICHISSEMENT EN GAZ ET SIMULTANÉMENT DE DÉPLACEMENT D'UN FLUIDE ET SYSTÈME POUR LE CONTRÔLE DE L'ENVIRONNEMENT CELLULAIRE SUR UNE PLAQUE DE CULTURE CELLULAIRE MULTIPUITS CORRESPONDANT**
VERFAHREN ZUR ANREICHERUNG MIT GAS UND GLEICHZEITIGER VERDRÄNGUNG EINES FLUIDS, UND ENTSPRECHENDES SYSTEM ZUR KONTROLLE DER ZELLUMGEBUNG AUF EINER MULTIWELL-ZELLKULTURPLATTE
METHOD FOR GAS ENRICHMENT AND SIMULTANEOUS MOVEMENT OF A FLUID AND SYSTEM FOR CONTROLLING THE CELLULAR ENVIRONMENT ON A CORRESPONDING MULTIWELL CELL CULTURE PLATE

(30) Priorité: 20.03.2019 FR 1902859
(43) Date de publication de la demande: 23.09.2020
(73) Titulaire: Cherry Biotech SAS, 35000 Rennes (FR)
(72) Inventeur: HOMS CORBERA, Antoni, 17220 Sant Feliu de Guixols (Girona) (ES); BONINSEGNA, Matteo, 59100 Prato (FR); VITAL, Théo, 35700 Rennes (FR)
(74) Mandataire: Vidon Brevets & Stratégie

(56) Documents cités:
- EP-A1- 2 623 587
- EP-A2- 0 263 634
- WO-A1-2004/027016
- WO-A1-2018/013646
- WO-A2-2018/136752

## Description

### Domaine technique

L'invention concerne le domaine de la culture *in vitro* de cellules, tissus ou organoïdes en plaques de culture notamment les plaques dites multipuits. En particulier, l'invention concerne le contrôle des conditions environnementales de culture *in vitro* au sein d'un espace confiné tel que des plaques de culture cellulaire. Plus particulièrement, l'invention concerne un procédé de déplacement d'un fluide et simultanément d'enrichissement en gaz d'un milieu de culture cellulaire liquide et un système de contrôle environnemental de culture cellulaire *in vitro* sur une plaque de culture cellulaire multipuits.

### Technique antérieure

Les cultures *in vitro* de cellules, ou d'assemblage cellulaire tel que la culture de tissus, de sphéroïdes ou encore plus récemment d'organoïdes, c'est-à-dire de structures multicellulaires tridimensionnelles qui reproduisent *in vitro* la micro-anatomie d'un organe, sont aujourd'hui de plus en plus utilisées comme outil d'évaluation toxicologique de substances, notamment pharmacologiques ou cosmétiques.

Ces modèles *in vitro* sont utilisés, notamment dans la recherche pharmaceutique, car ils représentent une alternative sérieuse aux modèles *in vivo,* c'est-à-dire à l'expérimentation animale, contre laquelle des pressions à la fois économiques et éthiques apparaissent au niveau international.

Ainsi, pour être en mesure de réaliser des expériences biologiques contrôlées à moyen et long terme pour analyser les composés thérapeutiques, cosmétiques ou toutes substances potentiellement dangereuses, il est essentiel de pouvoir contrôler les paramètres de culture cellulaire, notamment les changements environnementaux sélectifs induits, tout en surveillant l'activité de ces environnements cellulaires confinés, et ceci de manière automatisée.

En effet, la préparation d'échantillons moléculaires et cellulaires de haute qualité est importante pour diverses applications cliniques, de recherche et autres. Les échantillons *in vitro* qui présentent de près leurs caractéristiques *in vivo* peuvent potentiellement bénéficier à un large éventail d'applications moléculaires et cellulaires. La manipulation, la caractérisation, la culture et la visualisation de cellules ou d'autres matériaux biologiquement ou chimiquement actifs (comme par exemple des billes recouvertes de diverses molécules biologiques) sont de plus en plus appréciées dans les domaines de la découverte de médicaments, du diagnostic et de l'analyse des maladies, et de divers autres travaux thérapeutiques et expérimentaux.

La culture cellulaire microfluidique, entendue comme la culture de cellules dans des chambres reliées et alimentées par des microcanaux caractérisés par des volumes fluidiques compris entre 1 pL et 100 mL, est une technologie importante pour le dépistage des drogues, la culture tissulaire, le dépistage de la toxicité et la recherche biologique. Elle améliore les conditions de culture des cellules, fournit des données cellulaires de meilleure qualité, réduit la consommation de réactifs et les coûts.

Ce système automatisé de culture cellulaire microfluidique de contrôle de l'environnement de culture *in vitro* permet un contrôle fin des conditions de culture cellulaire, afin de ce rapprocher le plus possible des conditions *in vivo.* Ce système est une combinaison de milieux contrôlés perfusés de composition chimique, biochimique et gazeuse adéquate, et d'une température contrôlée. Ce système de culture cellulaire microfluidique permet de garantir le contrôle du stress de cisaillement des cellules cultivées, ainsi que l'évitement de fuites d'éléments biologiques et/ou de composés sensibles et/ou dangereux et/ou de contamination par des germes étrangers ou des particules indésirables des échantillons lors de l'exécution de protocoles expérimentaux.

L'outil standard actuel en matière de recherche analytique et de tests diagnostiques clinique utilisé en laboratoire est la plaque de culture à plusieurs puits, ou plaque de culture dites multipuits. Ces plaques multipuits sont utilisées depuis de nombreuses années pour la culture de cellules et de tissus.

Pour la protection des cultures cellulaires contre la contamination par des germes étrangers ou des particules indésirables, les plaques multipuits sont fermées par un couvercle assorti. Selon le type cellulaire ou les tissus en croissance dans les puits, il est nécessaire de régulièrement changer le milieu de culture des cellules, afin de renouveler les nutriments et/ou le traitement appliqué sur les cellules (Paul J.: Culture cellulaire et tissulaire. 5e éd.Livingston, Édimbourg, 1975). Le changement de milieu de culture cellulaire ou le traitement des cellules se fait généralement manuellement ce qui prend non seulement beaucoup de temps, mais également comporte un risque de contamination par des germes étrangers ou particules indésirables provenant de l'environnement extérieur (Freshney R. I. : Animal Cell Culture : A Pratique Approche. 2nd Ed. a.R. Liss, New York, 1987).

Les limites de l'utilisation actuelle de ces dispositifs incluent le fait que la culture à long terme de cellules implique un échange et un enrichissement continus du milieu cellulaire en utilisant des méthodes qui ont de nombreuses limitations dépendant de facteurs externes limitant les essais continus de matériel biologique dans la plaque de culture à plusieurs puits avec des environnements contrôlés à seulement quelques jours

La demande de brevet WO 2009089189 (MILLIPORE CORPORATION) décrit un système de culture cellulaire microfluidique comprenant entre autre une chambre microfluidique améliorant les conditions de culture cellulaire. Le déplacement sous pression des liquides vers la chambre microfluidique de culture cellulaire permet d'obtenir une grande précision, même pour de très petits volumes. La commutation du flux laminaire rapide entre cinq solutions d'entrée du flux, ainsi que les barrières de perfusion permettent un transport de masse continu sans contrainte de cisaillement sur les cellules. Ce système permet notamment d'effectuer des expériences d'analyse de cellules vivantes pendant plus de trois jours, mais pas plus de cinq jours sur la platine de n'importe quel microscope inversé standard. Ce système possède également un contrôle dynamique du débit du gaz et de la température.

Toutefois, ce système n'est pas sans inconvénients. En effet, la complexité élevée de ce système entraîne un taux de défaillance élevé de l'appareil construit. La perfusion du milieu d'enrichissement est obtenue par diffusion, ce qui limite l'enrichissement rapide en nutriments, ainsi que les posologies de composés à tester. L'enrichissement en gaz du milieu des cellules se fait par diffusion à travers un matériau poreux (tel que le silicium élastomère Polydiméthylsiloxane, PDMS) qui limite également l'efficacité et la force d'enrichissement des gaz et oblige à utiliser des matériaux qui peuvent potentiellement absorber les molécules essentielles étudiées pour leurs effets, telles que les composés pharmacologiques ou cosmétiques, présentes dans le milieu perfusé. Le système ne convient pas à la culture de grands ensembles biologiques tels que des biopsies de tissus en raison de ses dimensions, et la durée de la culture est limitée à 3 jours, maximum 5 jours.

De plus, ce système n'est pas entièrement compatible avec l'utilisation de plaques de culture multipuits standards.

Finalement, ce système manque de flexibilité pour interconnecter différentes cultures cellulaires et fourniture des milieux perfusés dans la plaque ou de choisir différentes configurations de puits portant des cellules ensemencées.

La demande de brevet WO2007092571 (WAFERGEN INC) décrit un dispositif de culture tissulaire isolée de l'environnement qui peut être utilisé pour la culture cellulaire. Ce dispositif de culture tissulaire comprend : une plaque multipuits comprenant une pluralité de puits, dans laquelle chaque puits est formé pour recevoir au moins deux tubes insérables, un premier collecteur d'alimentation d'éléments nutritifs comprenant une pluralité de conduites individuelles d'alimentation de puits, un collecteur placé au-dessus des puits et aligné de telle sorte que chaque conduite d'alimentation de puits s'étend jusqu'à une position adjacente au fond d'un puits, un deuxième collecteur d'élimination de déchets comprenant une pluralité de lignes individuelles d'élimination de déchets. Ce deuxième collecteur s'étend jusqu'à une position adjacente au fond du puits, où la conduite d'alimentation en déchets s'étend jusqu'à un point légèrement surélevé par rapport à la ligne d'alimentation du puits.

Dans certaines variantes, les puits peuvent inclure des orifices reliant les puits adjacents les uns aux autres, ce qui permet le passage de matériaux entre les puits individuels de la plaque.

Un couvercle à température contrôlée comprenant des entrées pour la diffusion de gaz est également décrit.

Les ports (connexion externe entrée/sortie) peuvent également comporter une ou plusieurs vannes de commande de l'ouverture/fermeture du port.

Ce système comprend certains inconvénients, notamment, le milieu de perfusion est alimenté de l'extérieur (actionneurs externes pour déplacer le liquide directement).

Le mélange de gaz est alimenté de l'extérieur pour l'enrichissement des milieux seulement par diffusion en surface à l'intérieur du puits. L'enrichissement en gaz en fournissant du gaz aux puits par des ports spécifiques ne convient pas aux dispositifs microfluidiques enrichis en gaz.

Les déchets sont aspirés de l'extérieur, indépendamment des actionneurs d'alimentation du fluide.

Gaz, liquides perfusés et déchets sont manipulés à l'aide d'actionneurs externes indépendants ce qui augmente la complexité du système.

Le mélange se fait dans un puits en agitant le puits vers l'extérieur ou en introduisant des agitateurs de type magnétiques, mécaniques, ce qui modifie potentiellement les conditions de position des cellules.

La manipulation des liquides, des réactifs ou de milieux, perfusés à l'intérieur n'est pas entièrement confinée ou isolée et peut fournir une contamination potentielle aux plaques multipuits et aux actionneurs externes surtout après leur utilisation.

Le document de brevet EP 0 263 364 A2 (SUZUKI SHOKAN KK) concerne un procédé d'alimentation en milieu de culture d'un récipient de culture cellulaire et les systèmes mettant en oeuvre ce procédé. Ce procédé comprend notamment l'injection de gaz frais dans des chambres de culture cellulaire du récipient. Cette injection de gaz se fait par le volume de gaz compris dans ces chambres.

Le document de brevet EP 2 623 587 (CELLIX LTD (IE)) concerne un système et un procédé de déplacement de milieu de culture cellulaire par différence de pression. Selon ce procédé, une fois que la pompe a aspiré une quantité de milieu frais prédéterminée, la pression s'égalise entre les deux récipients. Le liquide se déplace par aspiration de la pompe et diminution de la pression dans l'un des récipients.

Le document de brevet WO 2018/136752 (ESSEN BIOSCIENCE INC) concerne un dispositif permettant notamment de contrôler la circulation de fluides et l'environnement atmosphérique d'une culture cellulaire en microplaques. Le déplacement du fluide dans un circuit microfluidique, ainsi que le contrôle de la composition en gaz du fluide se font par contrôle de la pression au niveau du couvercle pneumatique, via un ou plusieurs raccords de commande pneumatique. L'injection de gaz pour créer la pression différentielle est effectuée par le dessus de puits d'une microplaque, c'est-à-dire par le volume de gaz.

Le document de brevet WO 2018/013646 (EMULATE INC) concerne des méthodes d'élimination des bulles dans un dispositif microfluidique sans que le flux n'ait besoin d'être arrêté. En particulier, WO 2018/013646 divulgue des méthodes qui combinent un contrôle de la pression et du débit.

Le document de brevet WO 2004/027016 (COOK UROLOGICAL, INC) concerne un incubateur à perfusion comprenant un ensemble de puits d'incubation. Une pompe péristaltique fait passer le milieu de culture à travers une unité de conditionnement avant d'alimenter l'ensemble des puits d'incubation avec ce milieu de culture conditionné.

Ainsi, les méthodes disponibles actuellement soit n'assurent pas l'isolation adéquate de l'extérieur, soit ont des limites en termes de polyvalence fluidique et de communication entre les puits, soit nécessitent des dispositions externes de manipulation des réactifs pendant l'expérimentation, soit ne sont pas capables de maintenir l'enrichissement gazeux du milieu, soit génèrent des forces et contraintes de cisaillement sur les cellules en culture et sur les ensembles cellulaires qui dépassent largement les niveaux physiologiques.

L'enrichissement en gaz est généralement difficile à réaliser dans les dispositifs microfluidiques et autres dispositifs de culture cellulaire où le contact de la surface liquide perfusée avec le gaz externe est limité ou inexistant et où les mécanismes de diffusion ne sont pas une solution efficace.

Il existe donc un besoin d'une technique et d'un appareil très polyvalents adaptable à la norme de la plaque de culture multipuits, mais sans s'y limiter, pour confiner dans des environnements de culture cellulaire hautement contrôlés, en termes de température, de composition du milieu, d'enrichissement de gaz, de déplacement de fluide. De plus, il existe un besoin d'isolation de l'extérieur de ces environnements contrôlés au sein des plaques de culture multipuits tout en permettant une observation optique et une communication sélective entre différents puits confinés sur la plaque multipuits lorsque nécessaire.

### Présentation de l'invention

L'invention répond à ce besoin en proposant un procédé de déplacement d'un fluide et simulténément d'enrichissement en gaz d'un milieu de culture cellulaire liquide.

Selon l'invention le procédé comprend :
- une injection d'un volume contrôlé d'un gaz ou mélange de gaz à l'intérieur d'au moins une chambre de pressurisation au moyen d'au moins un contrôleur de débit gazeux. Cette injection se fait par au moins une entrée de gaz dans un volume de liquide, l'injection produisant un bullage et une agitation du volume de milieu de culture cellulaire liquide;
- une accumulation du gaz ou mélange de gaz par flottabilité dans un espace hermétique formé par le volume de milieu de culture cellulaire liquide et ladite au moins une chambre de pressurisation, et
- une augmentation d'une pression dans ladite au moins une chambre de pressurisation jusqu'à atteindre une pression contrôlée suffisante inférieure ou égale à 10 bar. Cette augmentation produit un déplacement du volume de milieu de culture cellulaire liquide par au moins une sortie de fluide reliant le volume de milieu de culture cellulaire liquide et l'extérieur de ladite au moins une chambre de pressurisation.

Dans une variante, la chambre de pressurisation peut être par exemple un réservoir isolé, ou réservoir individuel isolé, aussi appelé réservoir étanche ou individuel indépendant, c'est-à-dire un réservoir isolé de milieux extérieurs non contrôlés. Ainsi, le procédé selon l'invention permet de créer un système basé sur la perfusion régulée de mélanges notamment gazeux. Ce système est utilisé non seulement dans une installation comprenant une plaque de culture multipuits, mais aussi pour déplacer un fluide, enrichir et contrôler le mélange gazeux des fluides dans toute installation microfluidique en utilisant le principe de la chambre pressurisée à bulles pour générer des flux microfluidiques.

Avantageusement, le procédé selon l'invention permet l'enrichissement en gaz des milieux perfusés dans un environnement totalement isolé pour les échantillons biologiques et les milieux utilisés.

Ainsi, il n'est pas nécessaire d'utiliser une structure d'incubateur pour garantir les conditions biologiques de vie dans le système fermé microfluidique ou fluidique selon l'invention.

Le procédé selon l'invention permet de maintenir une culture cellulaire, un tissu ou toute autre matière vivante en condition vivante grâce à un contrôle précis des paramètres biologiques vitaux tels que la composition des nutriments du milieu, le mélange gazeux et la température, et permet un contrôle précis du stress de cisaillement des cellules grâce à de faibles flux de perfusion pour éviter que la viabilité soit compromise.

Il est important de pouvoir contrôler les différents débits de gaz afin de pouvoir donner un pourcentage de débit pour chaque gaz tout en continuant à produire le débit total de sorte qu'en temps réel, le mélange de gaz puisse être modifié tout en produisant la même quantité de gaz et le débit fluidique souhaités.

Selon un aspect particulier de l'invention ledit au moins un gaz injecté est choisi parmi le O₂, CO₂, N₂ ou leurs mélanges.

Ce procédé permet de recréer différentes concentrations de gaz sur les milieux cellulaires pour mieux imiter les conditions physiologiques et pathologiques des tissus, organoïdes, sphéroïdes et cultures cellulaires dans la nature. De nombreux autres gaz et mélanges de gaz peuvent être utilisés.

Selon un autre aspect de l'invention le déplacement du volume de milieu de culture cellulaire liquide par ladite au moins une sortie de fluide reliant le volume de milieu de culture cellulaire liquide et l'extérieur de ladite au moins une chambre de pressurisation se fait vers un dispositif microfluidique.

Dans une variante, le volume de liquide peut être remplacé par un volume de gaz ou mélange de gaz une fois que la totalité du volume de liquide est déplacée. De manière avantageuse, le procédé peut ainsi être mis en oeuvre par un système comprenant une série de chambre de pressurisation montée en série ou en parallèle les unes des autres et dans lesquelles peuvent être déplacé successivement d'une chambre à l'autre un liquide et/ou un gaz, ou n'importe quel autre type de fluide.

L'invention concerne également un système de culture cellulaire. Ce sytème de culture cellulaire comprend :
- au moins une chambre de pressurisation comprenant un volume de milieu de culture cellulaire liquide. Cette chambre de pressurisation comprend en outre au moins une entrée de gaz et au moins une sortie de fluide reliant le volume de milieu de culture cellulaire liquide et l'extérieur de ladite au moins une chambre de pressurisation; et
- au moins un contrôleur de débit gazeux configuré pour injecter par l'entrée de gaz dans le volume de milieu de culture cellulaire liquide un volume contrôlé d'un gaz ou mélange de gaz à l'intérieur de ladite au moins une chambre de pressurisation.

Ce système en outre met en oeuvre le procédé selon l'invention, tel que décrit précédemment.

Selon un autre aspect de l'invention, le système comprend plusieurs chambres de pressurisation montées en série ou en parallèle les unes des autres et dans lesquelles le fluide est déplacé successivement d'une chambre à l'autre.

Selon un autre aspect de l'invention, ladite au moins une chambre de pressurisation est formée par un réservoir individuel isolé.

Le système de culture cellulaire selon l'invention est avantageusement compatible avec n'importe quel type de plaque multipuits jetables. Ces plaques multipuits sont un standard fortement utilisé dans les expériences pharmaceutiques, biotechnologiques et des sciences de la vie.

Ainsi, le système de culture cellulaire selon l'invention est simple d'utilisation quelque soit le mode de réalisation et peut être adapté facilement à n'importe quelles plaques multipuits standards. Les cellules mises en culture dans la plaque multipuits sont parfaitement isolées de l'environnement extérieur et les changements de milieu nutritif, de traitement, ne nécessitent plus une manipulation manuelle. Les paramètres de température et d'enrichissement en gaz sont contrôlés de manière automatisée ce qui permet de se passer d'appareil de type incubateur.

Le système de culture cellulaire selon l'invention permet de déplacer sélectivement des fluides d'un puits à un autre pour perfuser des milieux, appliquer un composé à un assemblage ou à une culture cellulaire donnée, ou permettre la communication entre différentes cellules assemblées ou en culture non organisée, pour recréer des communications multi organoïdes dans les systèmes biologiques.

Toutes les opérations effectuées garantissent l'isolement des composés, milieux et entités biologiques testés, des dispositifs de contrôle d'injection des gaz et liquides.

Le système selon l'invention permet de contrôler en temps réel les environnements de culture cellulaire (ou toute entité biologique : sphéroïdes, tissus, organoïdes, cellules à matrice extracellulaire, cellules à hydrogel...) comme par exemple en contrôlant la perfusion des milieux de culture à des valeurs données de pression/débit, et en option la sélection de liquide et de température. La manipulation est entièrement confinée/isolée des réactifs perfusés à l'intérieur de l'appareil (système complet seulement) contrôlé par l'application de pressions de gaz externes ou l'introduction fluidiquement isolée de liquides de thermalisation.

Ce système permet également une observation microscopique des échantillons vivants en temps réel pendant l'expérience.

Selon une autre caractéristique de l'invention, le système de culture cellulaire comprend en outre :
- une plaque de culture cellulaire multipuits;
- un dispositif destiné à être adapté sur la plaque de culture cellulaire multipuits, ce dispositif comprenant au moins un circuit microfluidique (A) comprenant au moins un canal microfluidique formé dans au moins une plaque microfluidique intégrée, ce circuit microfluidique (A) s'étendant depuis au moins un orifice de raccordement ou au moins un conduit de raccordement fluidique vers un collecteur. Le collecteur comprend en outre au moins une cavité, ladite au moins une cavité formant avec au moins un puits de la plaque de culture cellulaire multipuits ladite au moins une chambre de pressurisation. En outre, le collecteur comprend au moins un ajutage ou au moins un orifice ou au moins un conduit, ledit au moins un ajutage ou au moins un orifice ou au moins un conduit prolongeant ledit au moins un canal microfluidique, et formant ledit au moins un circuit microfluidique (A) complet lorsque le dispositif est combiné à la plaque de culture cellulaire multipuits.

Selon un autre aspect de l'invention, le dispositif comprend au moins un autre circuit microfluidique parallèle intégré (B), indépendant dudit au moins un circuit microfluidique (A), ledit au moins un autre circuit microfluidique parallèle intégré (B) présentant au moins un canal microfluidique formé dans au moins une autre plaque microfluidique parallèle indépendante de la plaque microfluidique.

Différentes plaques de canaux microfluidiques peuvent être placées dans le dispositif selon l'invention afin d'augmenter encore le contrôle de l'environnement biologique de culture cellulaire et des fluides perfusés sur l'appareil si nécessaire.

Selon une autre caractéristique de l'invention, un fluide injecté s'écoule depuis ledit au moins un orifice de raccordement ou ledit au moins un conduit de raccordement fluidique à l'intérieur dudit au moins un circuit microfluidique (A) vers le collecteur et vers la plaque de culture cellulaire multipuits.

Ainsi, un fluide (gaz ou liquide) est injecté au niveau d'au moins un orifice ou au moins un conduit de raccordement fluidique et s'écoule ensuite à travers au moins un circuit microfluidique vers au moins un ajutage ou au moins un orifice ou au moins un conduit du collecteur. Le fluide se dirige donc vers une chambre, dite chambre de pressurisation, formée par une cavité ou espace vide du collecteur et un puits de la plaque multipuits sur lequel ledit dispositif est adapté. Dans une variante, le fluide peut être simplement évacué vers l'extérieur du dispositif.

Ainsi, l'introduction d'un fluide (liquide ou gaz) depuis les ajutages ou les orifices ou les conduits vers les puits de la plaque multipuits contenant du liquide, se fait jusqu'à un niveau fermant ledit ajutages ou orifices ou conduits, permet d'augmenter la pression à l'intérieur du puits et permet non seulement un déplacement fluidique à travers d'autre ajutages ou orifices ou conduits présent dans les puits de la plaque multipuits soumis à une pression externe inférieure à la pression exercée à l'intérieur du puits, mais également, grâce à un effet de bulle et d'agitation associée du liquide, d'enrichir par exemple le milieu de culture et/ou les liquides présents dans les puits ou injectés dans le circuit microfluidique.

Selon un autre caractéristique de l'invention un liquide de thermalisation injecté s'écoule depuis ledit au moins un orifice de raccordement ou ledit au moins un conduit de raccordement fluidique à l'intérieur dudit au moins un circuit microfluidique parallèle intégré (B).

Avantageusement, ce fluide est isolé fluidiquement des autres circuits microfluidiques du dispositif.

Ainsi, il est possible d'intégrer également le contrôle de la température de l'environnement biologique de culture cellulaire en faisant circuler une ou plusieurs solutions de thermalisation dans le dispositif selon l'invention (isolées des composants testés, des milieux et des entités biologiques). Il peut également être possible de maintenir différentes parties de la plaque multipuits à différentes températures garantissant l'intégrité des échantillons et des composés pour certaines expériences (les médicaments sont parfois conservés à des températures inférieures avant application).

### Liste des figures

D'autres buts, caractéristiques et avantages de l'invention apparaîtront plus clairement à la lecture de la description suivante, donnée à titre de simple exemple illustratif, et non limitatif, en relation avec les figures, parmi lesquelles :
**[****fig. 1****]** : illustre un schéma en vue explosée de la structure du dispositif selon un mode de réalisation de l'invention;
**[****fig. 2****]** : présente une vue schématique explosée de la structure du dispositif selon un autre mode de réalisation de l'invention;
**[****fig. 3****]** : présente un schéma d'une vue en coupe d'une partie du dispositif selon l'un quelconque des modes de réalisation en lien avec les **figures 1** et **2****;**
**[****fig. 4A****]** : illustre une vue schématique explosée du dispositif selon le mode de réalisation de la **figure 2** avec une plaque multipuits;
**[****fig. 4B****]** : illustre une vue schématique du dispositif selon l'un quelconque des modes de réalisation en lien avec les **figures 1** et **2** avec une plaque multipuits;
**[****fig. 5****]** : illustre une vue d'un assemblage d'une plaque multipuits avec le dispositif selon l'un quelconque des modes de réalisation en lien avec les **figures 1** et **2** une fois raccordés au contrôleur de débit gazeux et de fluides de thermalisation externe et placé dans un microscope pour l'imagerie des cellules en temps réel;
**[****fig. 6A****]** : présente un schéma d'un exemple d'une chambre de pressurisation mettant en oeuvre le procédé selon l'invention;
**[****fig. 6B****]** : présente un schéma d'un exemple d'une chambre de pressurisation mettant en oeuvre le procédé selon l'invention lorsque celle-ci est reliée au dispositif selon l'un quelconque des modes de réalisation en lien avec les **figures 1** et **2****.**

### Description détaillée de modes de réalisation de l'invention

Le principe général de l'invention consiste à confiner et contrôler de manière temporelle et spatiale les propriétés chimiques, biochimiques et physiques, telles que les contraintes de cisaillement induites ou les températures locales, des environnements biologiques au sein des dispositifs comme par exemple des dispositifs microfluidiques et au sein des puits de plaques de culture cellulaire à plusieurs puits, aussi appelées plaques de culture multipuits, au moyen d'un dispositif spécialement conçu. L'invention est utile pour effectuer des essais biologiques contrôlés dans les domaines de la découverte et des essais pharmacologiques et biotechnologiques, de la différenciation cellulaire contrôlée, des essais thérapeutiques et cosmétiques, de la recherche et des essais de composés et des essais personnalisés, ainsi que dans toutes expériences en sciences du vivant nécessitant un contrôle précis des conditions environnementales des cellules, tissus ou organoïdes.

Plus particulièrement, l'invention propose simultanément d'enrichir un liquide, comme par exemple un milieu de culture cellulaire, en gaz tout en le déplaçant vers un dispositif microfluidique. Cette étape préalable d'enrichissement en gaz d'un milieu de culture permet notamment de maintenir les conditions de culture cellulaire sur le long terme.

Par la suite, par soucis de simplification, on entend par « culture cellulaire » une culture de cellules non organisées ou organisée, c'est-à-dire un assemblage cellulaire comme par exemple les tissus, les sphéroïdes ou organoïdes. Ces cellules peuvent être issues par exemple d'animaux tel que l'Homme, la souris, ou de végétaux.

Par « plaque multipuits » on entend des plaques standards comprenant au moins six puits dans lesquels un milieu de culture nutritif spécifique, certains composés chimique ou biochimique, au type cellulaire étudié est introduit, ainsi que des cellules à cultiver.

Par « microfluidique » on entend des canaux de sections transversales entre 1µm² et 10mm².

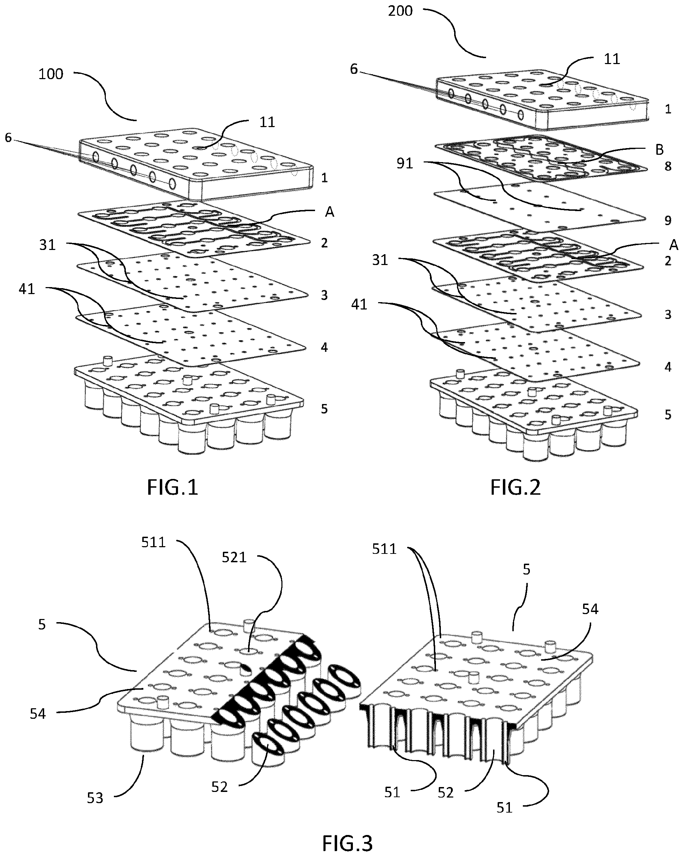

On présente désormais en lien avec la **figure 1** **la** structure du dispositif, selon un premier mode de réalisation de l'invention.

Le dispositif 100 de forme sélectionnées parmi les formes cubiques, rectangulaire, mais de préférence de forme sensiblement parallélépipédique comprend un couvercle 1, ainsi qu'une partie, appelée collecteur 5, comprenant un ensemble d'ajutages ou d'orifices ou de conduits formant la base de ce dispositif.

Le couvercle 1 comprend au niveau de deux faces latérales opposées un ou plusieurs orifice(s) de raccordement 6 ou conduit(s) de raccordement fluidique (non représentés) permettant la fixation d'au moins un connecteur CNT de fluide (non représenté) provenant d'un contrôleur de débit gazeux fournissant de manière externe un fluide tel qu'un gaz ou un liquide, pour un contrôle externe de la pression du fluide et/ou de son débit (ou flux) en temps réel. Ce couvercle 1 peut être complètement opaque ou, dans une variante, permettre le passage de la lumière afin d'obtenir une image optique en temps réel des échantillons biologiques.

Dans cette variante, au niveau de la surface supérieure externe du couvercle 1, plusieurs zones traversantes 11 permettent le passage de la lumière et ainsi une visualisation en microscopie ou par d'autres moyens nécessaires à l'obtention d'images en temps réel des échantillons biologiques. Le couvercle 1 est préférentiellement de surface plane au moins sur sa face inférieure. Il peut être fabriqué avec différents matériaux incluant, mais sans limitation, des polymères tels que PMMA (poly(méthacrylate de méthyle)), COP, PS (polystyrène), PEEK (polyétheréthercétone) ou PC (polycarbonate).

Une plaque microfluidique 2 est ensuite fixée entre les surfaces planes de la partie inférieure du couvercle 1 et de la partie supérieure d'une plaque de liaison 3 qui garantit l'étanchéité sélective des canaux et des structures microfluidiques. Cette plaque microfluidique 2 est préférentiellement en ruban adhésif double face, découpé de manière à définir des canaux microfluidiques reliant les différents puits de la plaque de culture multipuits 7 (non représentée) aux ajutages ou orifices ou conduits du collecteur 5 et aux orifices de raccordement 6 ou conduits de raccordement fluidique du couvercle 1 correspondants. Alternativement, la plaque microfluidique est construite selon une technique de collage de polymère. Cette plaque microfluidique 2 définie les connexions fluidiques gazeuses ou liquides du dispositif microfluidique 100.

Dans une variante, le dispositif 100 peut être réalisé de différentes manières, par exemple la plaque microfluidique 2 est construite dans le couvercle 1, puis collée ou liée à la plaque de liaison 3 ou à la plaque de liaison 4 ou au collecteur 5 directement.

La plaque de liaison 3, de surface sensiblement plane, permet de faciliter la fixation de la plaque microfluidique 2 à la plaque de fixation 4 et garantit l'étanchéité sélective des canaux microfluidiques. Ainsi, le couvercle 1 peut être fixé par l'intermédiaire de la plaque microfluidique 2, de la plaque de liaison 3 et de la plaque de fixation 4 au collecteur 5, et par conséquent créer un dispositif fluidique à assembler ultérieurement à une plaque multipuits 7 (non représentée) pour générer la structure fluidique complète. Cette structure en sandwich et ses variantes sont réalisées pour garantir le bon assemblage des différentes pièces en une structure microfluidique assurant une communication sélective avec un fournisseur de gaz extérieur contrôlé, un mouvement des fluides entre les puits la plaque multipuits 7 (non représentée) tout en évitant une contamination indésirable et la circulation externe des liquides.

La surface de la plaque de liaison 3 est préférentiellement transparente pour une application où la lumière doit passer des zones traversantes supérieures 11 du couvercle 1 au fond des puits de la plaque multipuits 7 pour l'imagerie microscopique par exemple. De manière préférentielle, les matériaux utilisés pour la fabrication de la plaque de liaison 3 sont choisis parmi les composés COP, COC, verre ou autres matériaux transparents. La plaque de liaison 3 est traversée par une pluralité d'orifices 31 permettant de relier les canaux microfluidiques de la plaque microfluidiques 2 aux canaux 51 représentés en **figure 3** du collecteur 5 correspondants.

Dans une variante, le dispositif 100 peut être réalisé de différentes manières, par exemple une plaque comme la plaque de liaison 3 pourrait être utilisé entre le couvercle 1 et plaque microfluidique 2 .

Afin de fixer l'ensemble couvercle 1, plaque microfluidique 2, plaque de liaison 3 au collecteur 5, une plaque de fixation 4 préférentiellement en ruban adhésif double face est utilisée. Cette plaque de fixation 4 permet également une bonne communication entre les puits de la plaque de culture multipuits 7 à travers une pluralité d'orifices 41 reliant les canaux microfluidiques définis par la plaque microfluidique 2 aux ajutages ou aux orifices ou aux conduits 51 du collecteur 5. Les orifices 41 sont en vis-à-vis des orifices 31.

Alternativement, cette plaque de fixation 4 peut être éliminée et la plaque de liaison 3 est alors fixée directement sur le collecteur 5. Par exemple, les différents éléments sont fixés les uns au autres par utilisation d'une technique de collage de polymères.

Le collecteur 5 est une structure en matériau flexible, comme par exemple un matériau élastomère tel que le polyuréthane ou le silicone, définissant des ajutages ou orifices ou conduits microfluidiques 51 et une cavité ou espace vide 52 atteignant une profondeur définie dans la plaque de culture multipuits 7, tout en scellant un circuit fluidique A. Ce circuit fluidique A est défini par les canaux microfluidiques de la plaque microfluidique 2, les orifices 31 de la plaque de liaison 3, les orifices 41 de la plaque de fixation 4 et les ajutages ou orifices ou conduits 51 du collecteur 5. La flexibilité du collecteur 5 facilité l'assemblage du dispositif aux puits de la plaque de culture multipuits 7 et garantit l'étanchéité de la structure résultante.

Alternativement, le collecteur 5 est une structure en matériau rigide, comme par exemple un polymère tel que PMMA (poly(méthacrylate de méthyle)), COP, PS (polystyrène), PEEK (polyétheréthercétone) ou PC (polycarbonate), définissant des ajutages ou des orifices ou des conduits microfluidiques 51 et une cavité ou espace vide 52 atteignant une profondeur définie dans la plaque de culture multipuits 7, tout en scellant un circuit fluidique A. Le dispositif est assemblé aux puits de la plaque de culture multipuits 7 et garantit l'étanchéité de la structure résultante par exemple par l'intermédiaire d'un ou plusieurs joint/s torique ou structure équivalente, ou encore par collage.

On présente désormais en lien avec la **figure 2** la structure du dispositif selon un autre mode de réalisation de l'invention.

Dans ce mode de réalisation particulier, une autre plaque microfluidique 8 conçue pour être indépendante et non reliée au circuit A de la plaque microfluidique 2 est ajoutée dans le but de générer une variation de l'invention originale dans laquelle une solution de thermalisation, c'est-à-dire un liquide capable de transporter la chaleur, est mise en circulation pour contrôler la température du dispositif 200. Un dispositif externe de chauffage ou de refroidissement automatique du liquide et des mécanismes de pompage sont alors nécessaire pour un contrôle en temps réel de la température.

Cette plaque microfluidique 8 peut être créée comme les autres plaques fluidiques 2 préférentiellement à l'aide d'un ruban double face définissant des canaux microfluidiques ou en l'incorporant directement dans la structure du couvercle 1. Cette plaque microfluidique 8 est insérée entre le couvercle 1 et la plaque microfluidique 2, ce qui permet entre autre la liaison de la plaque microfluidique 2 avec le couvercle 1.

Dans une variante, la plaque microfluidique 8 peut être insérée de différentes manières et à différents niveaux de la structure en sandwich, comme il peut en être facilement déduit.

Une plaque de liaison 9 de surface sensiblement plane est utilisée pour faciliter la connexion de la plaque microfluidique 8 et de la plaque microfluidique 2 avec le couvercle 1. La plaque de liaison 9 est préférentiellement transparente et fabriquée dans des matériaux choisis parmi le COP, COC, verre ou autre matériau transparent, pour une application où la lumière doit traverser les zones traversantes supérieures 11 vers le bas des puits de la plaque multiples 7 à travers de la cavité ou de l'espace vide 52 **(****figure 3****)** pour l'imagerie microscopique par exemple. Dans une variante, la plaque de liaison 9 comprend également des orifices 91 permettant de relier les canaux microfluidiques de la plaque 8 vers certains orifices 31 et 41 afin de relier certains ajutages ou orifices ou conduits microfluidiques 51 aux canaux microfluidiques de la plaque 8. Ainsi, un deuxième circuit microfluidique parallèle B et indépendant de celui formé par la plaque 2 peut être formé. Ce circuit parallèle B permet notamment de réguler la température de la plaque multipuits 7.

Dans un mode de réalisation particulier, la plaque microfluidique 8 est dépendante de la plaque microfluidique 2 et les circuit microfluidique A et B sont également dépendants ou indépendants.

Comme variation, un ou plusieurs fluide(s) de thermalisation à température contrôlée extérieurement peut être envoyé dans des canaux microfluidiques parallèles sélectionnés différents ou similaires afin de réguler la température de l'ensemble des puits de la plaque multipuits. Dans une autre variation, la température de seulement certains puits sélectionnés est régulée à l'aide de l'injection d'un ou plusieurs liquide(s) de thermalisation dans des canaux microfluidiques sélectionnés. Dans une autre variante, on intègre des fluides spécifiques pour perfuser les solutés de thermodurcissables et contrôler la ou les température(s) du dispositif ou de certaines ses parties.

Comme variation des deux modes de réalisation précédents, des filtres hydrophobes séparant les phases gazeuses et liquides peuvent être mis en place au niveau des entrées et sorties de gaz, évitant ainsi l'entrée ou la sortie de liquide du dispositif selon l'invention et de la plaque multipuits 7 une fois assemblés.

Dans une autre variante, la structure du dispositif selon le mode de réalisation en lien avec les **figures 1** ou **2** comprend un assemblage en sandwich d'une pile de plaques microfluidiques 2 ou 8, avec des structures microfluidiques égales ou différentes, en alternance avec des plaque de liaison 3 ou 9 pour des conceptions de circuit microfluidiques plus complexes ou alternativement tridimensionnelles.

Dans une variante, le dispositif comprend des orifices ou des conduits pour la récupération sélective des fluides pendant le fonctionnement de l'appareil (à des fins d'échantillonnage analytique).

Dans une autre variante, des dispositifs de détection et de biodétection sont intégrés dans le dispositif selon l'invention pour analyser en temps réel l'échantillon, les fluides perfusés ou les fluides résiduaires résultants (ceux-ci peuvent être de toute nature: électrique, optique, mécanique, ou autre). Ces dispositifs peuvent par exemple contrôler différents paramètres des expériences associées (pH, température, potassium, électricité, etc.)

Les éléments 1 à 5 dans le mode de réalisation de la **figure 1**, ou 1 à 9 dans le mode de réalisation de la **figure 2** doivent être assemblés de manière permanente pour former le dispositif selon l'invention.

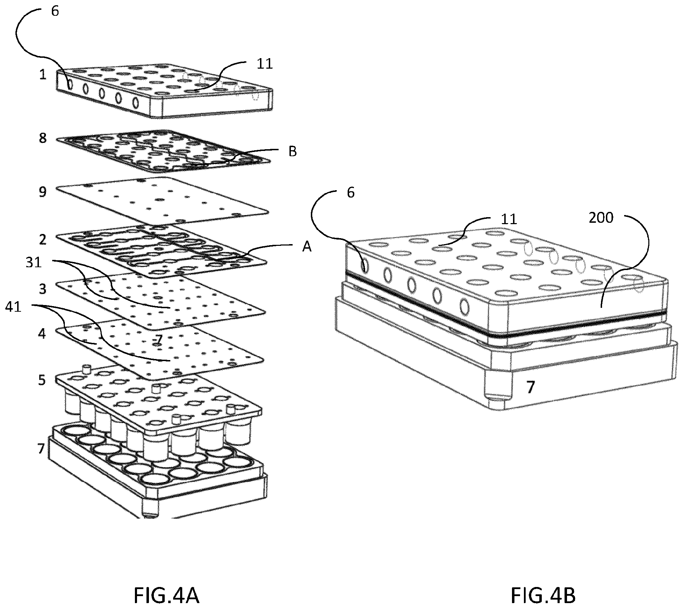

Le dispositif selon l'invention est ensuite monté au niveau de la partie supérieure d'une plaque de culture multipuits standard 7, comme représenté par la suite en lien avec les **figures 4A** à **4B** et comprimé par des moyens externes, afin que le système dispositif selon l'invention - plaque de culture multipuits 7 soit complètement étanche, à l'exception des orifices de raccordement 6 ou des conduits de raccordement fluidiques .

On présente en lien avec la **figure 3** un schéma d'une vue en coupe de la structure du collecteur 5 du dispositif selon l'un quelconque des modes de réalisation selon la **figure 1** et **2****.**

Le collecteur 5 comprend une surface plane 54 sensiblement parallélépipédique recouvrant des structures de liaison 53 comprenant des ajutages ou des orifices ou des conduits microfluidiques 51, avec une cavité ou un espace vide 52 . La cavité ou espace vide 52 peut par exemple être de forme sensiblement cylindrique, parallélépipédique ou cubique.

La surface plane 54 comprend des orifices 511 permettant la liaison entre les canaux microfluidiques des plaques 2 et/ou 8 aux ajutages ou aux orifices ou aux conduits microfluidiques 51. Les orifices 521 permettent de laisser passer la lumière vers le fond du puits lorsque de l'imagerie microscopique est nécessaire.

On présente désormais en lien avec les **figures 4A à 4B****,** un exemple d'assemblage du dispositif selon l'un quelconque des modes de réalisation selon les **figures 1** et **2** sur une plaque multipuits.

La **figure 4A** présente un schéma éclaté d'un assemblage du dispositif 200 sur une plaque multipuits 7.

La **figure 4B** représente un schéma d'assemblage du dispositif selon l'un quelconque des modes de réalisation selon les **figures 1** et **2** sur une plaque multipuits 7.

Avant cet assemblage, les échantillons sont placés dans la plaque multipuits 7, ainsi que les réactifs, avant de la fermer avec le dispositif assemblé. Les milieux, les échantillons, les cultures cellulaires et les réactifs sont placés dans les puits de la plaque multipuits avant l'expérimentation (comme c'est le cas actuellement dans les laboratoires pharmaceutiques, biotechnologiques et biologiques) et restent isolés pendant toute son utilisation après l'assemblage du dispositif selon l'invention.

Dans une variante un ou plusieurs composé(s) sont perfusés sélectivement dans des puits ou réservoirs individuels indépendants.

Une fois assemblés, les deux parties sont scellées et reliées à l'extérieur uniquement par les orifices de raccordement 6 ou des conduits de raccordement fluidique. Les réactifs et les échantillons biologiques sont complètement isolés de l'extérieur puisque les orifices sont utilisés soit pour faire circuler les fluides, gaz ou liquide (qui sont préférentiellement préfiltrés pour éviter la contamination). Les solutions thermalisantes circulant dans des circuits microfluidiques indépendants ne doivent jamais être en contact avec les réactifs, ni les échantillons biologiques.

Dans une variante, le dispositif comprend une ou plusieurs ajutages ou orifices ou conduits microfluidiques 51 avec des hauteurs différentes pour contrôler, par combinaison avec les pressions et/ou les flux de gaz données, quel liquide est perfusé dans le ou les réservoirs qui contiennent le matériel biologique. Ces ajutages ou orifices ou conduits microfluidiques 51 sont conçus pour contrôler dans le temps l'application sélective d'une dose donnée de composés spécifiques tels que des médicaments ou des particules toxiques se mélangeant avec les milieux habituels de maintien de la vie pour le matériel biologique.

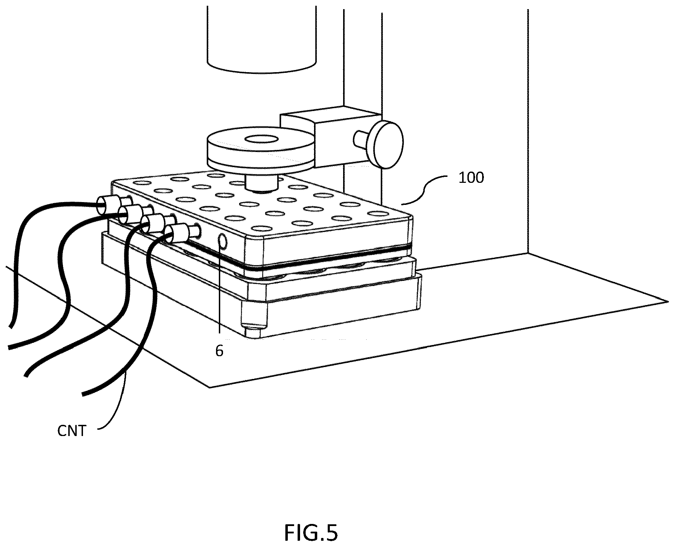

On présente désormais en lien avec la **figures 5** le dispositif selon l'un quelconque des modes de réalisation des **figures 1** et **2** assemblé à une plaque de culture multipuits chargées de réactifs et de matériel biologique, une fois raccordés au contrôleur de débits gazeux et de fluides de thermalisation externe et placé dans un microscope pour l'imagerie des cellules en temps réel.

La **figure 5** présente une installation commandée par un système de contrôle de la pression et/ou du flux (ou débit) d'un gaz ou mélange gazeux commandé par ordinateur et observé à l'aide d'un microscope inversé par exemple. L'installation comprend entre autre l'assemblage dispositif 100 ou 200 selon l'invention /plaque multipuits 7, ainsi qu'une alimentation en gaz externe avec contrôle en temps réel du mélange de plusieurs gaz (non représentée), un régulateur (non représenté) de flux et/ou de pression précis et en temps réel pour réguler le flux du gaz ou mélange gazeux, un ou plusieurs connecteur(s) CNT pour l'application de fluide, gaz ou liquide, ou d'un mélange de gaz contrôlé de différents pourcentages de O₂, CO₂ et N₂ contrôlé en temps réel, par exemple.

Dans l'installation, la température de l'étage du microscope est également contrôlée pour maintenir la bonne température au fond de l'assemblage afin de garantir la survie des cellules pendant de longues périodes de temps.

Dans une configuration alternative, la température est entièrement contrôlée par la solution de thermalisation qui s'écoule à travers le dispositif selon l'invention.

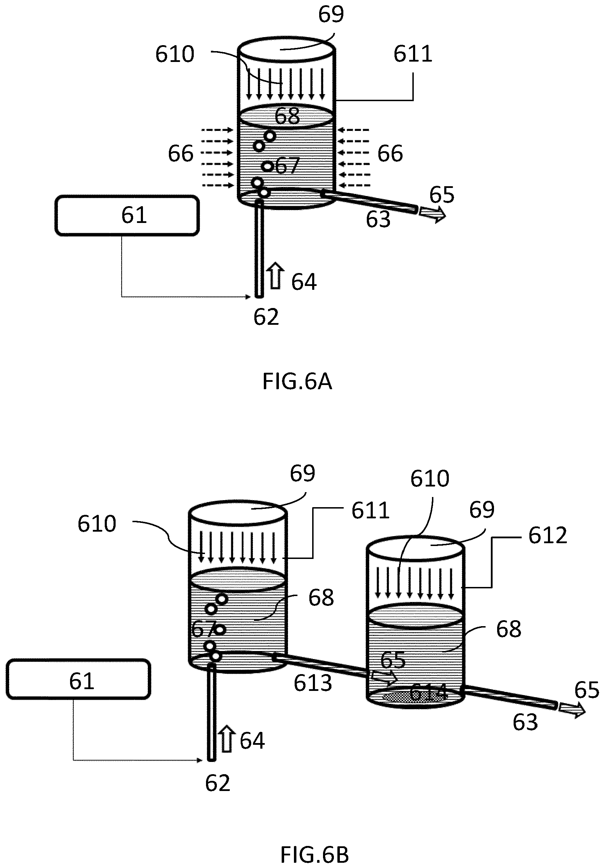

On présente désormais en relation avec les **figures 6A et 6B** un schéma d'un exemple d'une chambre de pressurisation mettant en oeuvre le procédé selon l'invention **(****figure 6A****)** lorsque celle-ci est associée à un contrôleur de débits gazeux. Dans un mode de réalisation particulier de l'invention elle peut également être reliée au dispositif selon l'un quelconque des modes de réalisation en lien avec les **figures 1** et **2** **(****figure 6B****).**

Par chambre de pressurisation, on entend par exemple un réservoir isolé qui pourrait être connecté à n'importe quel appareil et plus particulièrement à tout dispositif microfluidique aux fins de l'invention, pouvant le cas échéant être associé à un contrôleur de débit gazeux, ou bien l'association du dispositif selon l'invention avec une plaque multipuits au moyen d'un dispositif d'adaptation conçu à cet effet comme celui présenté dans cette invention. La chambre de pressurisation étant formée dans ce cas par le collecteur 5 et le puits de la plaque une fois que le dispositif est placé sur et scelle la plaque multipuits 7. Dans un autre exemple un ou plusieurs réservoir(s) isolé(s) formant une chambre de pressurisation peuvent être monté(s) en série ou en parallèle d'un ou plusieurs dispositifs selon l'invention.

En particulier la **figure 6A** présente un schéma du procédé de contrôle du déplacement et de l'enrichissement en gaz d'un milieu cellulaire perfusé pour la culture à long terme de tissus, cellules, sphéroïdes ou organoïdes dans des dispositifs hermétiques fermés. Ce procédé peut être mise en oeuvre par des puces microfluidiques, des réservoirs isolés de milieux extérieurs non contrôlés pouvant être par exemple associé à un contrôleur de débits gazeux ou encore au sein de plaques à plusieurs puits scellées avec un dispositif selon l'invention avec leurs puits isolés de milieux extérieurs non contrôlés. Ce procédé peut également être mis en oeuvre par un système comprenant d'une part un réservoir isolé, aussi appelé chambre de pressurisation, associé à un contrôleur de débits gazeux.

Un contrôleur de débits de gaz et/ou mélange gazeux 61, aussi appelé contrôleur de débit gazeux, permet de contrôler la composition en gaz, le débit (ou flux) gazeux, ainsi que la pression du gaz ou mélange de gaz.

Un gaz ou un mélange gazeux est injecté à travers une entrée de gaz 62, prenant la forme d'un orifice ou d'un conduit, pour une injection dans le réservoir étanche, aussi appelé chambre de pressurisation 611, au niveau d'un volume liquide 68 contenus dans cette chambre. Le réservoir étanche comprend un nombre donné d'entrées de gaz 62 et de sorties de fluide 63. Le volume de liquide 68, peut être par exemple un milieu de culture cellulaire, un traitement etc.... La direction du flux gazeux 64 s'étend depuis le contrôleur de débit gazeux 61 vers le volume de liquide 68.

Au fur et à mesure de l'injection en gaz, des bulles de gaz 67 remontent vers le volume de gaz 69 à travers le volume de liquide 68 tout en perturbant le liquide et en facilitant le mélange des liquides et l'absorption des gaz. Le volume de gaz 69 est scellé par le liquide 68 et le réservoir (ou chambre de pressurisation) 611. Le volume de liquide 68 et le réservoir (ou chambre de pressurisation) 611 forme ainsi un espace hermétique. La pression à l'intérieur de la chambre de pressurisation 611 augmente par effet de flottabilité du gaz, formant ainsi une contrepression 610 produite par le gaz s'accumulant dans le volume de gaz 69 et poussant le volume de liquide 68. La pression augmente ainsi jusqu'à atteindre une pression contrôlée suffisante inférieure ou égale à 10 bar.

Ce volume de liquide est ensuite évacué à travers une sortie de fluide 63, prenant la forme d'un orifice ou d'un conduit, le fluide pouvant être dans un premier temps un liquide, puis une fois que le liquide est évacué un gaz. La direction 65 du liquide, ou du gaz lorsqu'il n'y a plus de liquide dans la chambre de pressurisation, s'étend depuis la chambre de pressurisation 611 vers l'extérieur de la chambre.

Dans une variante, un système de chauffage et/ou refroidissement externe 66 permet de réguler la température de la chambre de pressurisation 611. Ce système peut être conçu pour fournir une convection liquide supplémentaire dans le réservoir, facilitant ainsi l'enrichissement en gaz et l'agitation du liquide.

La **figure 6B** présente un schéma d'un exemple d'une chambre de pressurisation mettant en oeuvre le procédé selon l'invention lorsque celle-ci est reliée au dispositif microfluidique selon l'un quelconque des modes de réalisation en lien avec les **figures 1** et **2****.** En particulier, la **figure 6B** présente un schéma d'un procédé pour déplacer une quantité contrôlée en temps réel de milieux de culture cellulaire enrichie d'un mélange gazeux contrôlé en temps réel pour la culture à long terme de matériel biologique (où le long terme dure entre 1 jour et 60 jours) dans des dispositifs hermétiques fermés tels que des puces microfluidiques, des réservoirs isolés des environnements externes non contrôlés ou des plaques à plusieurs puits scellées par le dispositif selon l'invention avec leurs puits isolés des environnements externes non contrôlés.

Dans ce mode de réalisation le volume de liquide 68 est évacué vers une entrée de fluide 613 d'un réservoir étanche 612 qui comprend un nombre donné d'entrées de fluide 613 et de sorties de fluide 63, prenant la forme d'un orifice ou d'un conduit. Dans ce réservoir 612 du matériel biologique 614 comme par exemple cellules, tissus, organoïdes ou sphéroïdes est mis en culture. La pression à l'intérieur du réservoir 612 augmente formant une contrepression 610 qui pousse sur le volume de liquide 68 qui est ensuite évacué vers des sorties 63 selon la direction 65.

Ce système de perfusion est basé sur des chambres pressurisées 611 ou 612 avec des ouvertures immergées dans un milieu liquide (au moins deux provenant de n'importe quel côté, de la partie inférieure ou supérieure) qui peut être utilisé sélectivement pour apporter une composition contrôlée de gaz pour effectuer simultanément un enrichissement sélectif du milieu en le poussant et en l'agitant par effet de bullage et accumulation (formation de pression) dans un espace supérieur du milieu gazeux. En d'autres termes, l'augmentation forcée de la pression du gaz dans la chambre de pressurisation 611, 612 permet d'enrichir le milieu de culture en gaz par l'effet de bullage du gaz sur le liquide tout en déplaçant le liquide dans la direction souhaitée en même temps.

Toutes les opérations fluidiques et les opérations d'enrichissement de gaz sont effectuées en contrôlant la pression et/ou le flux du fluide (gaz) et/ou du mélange de fluide (mélange de gaz) appliquée aux différentes entrées de gaz 62.

## Revendications

1. Procédé de déplacement d'un fluide et simultanément d'enrichissement en gaz d'un milieu de culture cellulaire liquide, **caractérisé en ce qu'**il comprend :
- une injection d'un volume contrôlé d'un gaz ou mélange de gaz à l'intérieur d'au moins une chambre de pressurisation (611, 612) au moyen d'au moins un contrôleur de débit gazeux (61), ladite injection se fait par au moins une entrée de gaz (62) dans un volume de liquide (68), ladite injection produisant un bullage et une agitation dudit volume de milieu de culture cellulaire liquide (68) ;
- une accumulation dudit gaz ou mélange de gaz par flottabilité dans un espace hermétique formé par ledit volume de milieu de culture cellulaire liquide (68) et ladite au moins une chambre de pressurisation (611, 612), et
- une augmentation d'une pression dans ladite au moins une chambre de pressurisation (611, 612) jusqu'à atteindre une pression contrôlée suffisante inférieure ou égale à 10 bar, ladite augmentation produisant un déplacement dudit volume de milieu de culture cellulaire liquide (68) par au moins une sortie de fluide (63) reliant ledit volume de milieu de culture cellulaire liquide (68) et l'extérieur de ladite au moins une chambre de pressurisation (611, 612).

2. Procédé de déplacement d'un fluide et simultanément d'enrichissement en gaz d'un milieu de culture cellulaire liquide selon la revendication 1, **caractérisé en ce que** ledit au moins un gaz injecté est choisi parmi le O₂, CO₂, N₂ ou leurs mélanges.

3. Procédé de déplacement d'un fluide et simultanément d'enrichissement en gaz d'un milieu de culture cellulaire liquide selon l'une quelconque des revendications 1 à 2, **caractérisé en ce que** ledit déplacement dudit volume de milieu de culture cellulaire liquide (68) par ladite au moins une sortie de fluide (63) reliant ledit volume de milieu de culture cellulaire liquide (68) et l'extérieur de ladite au moins une chambre de pressurisation (611, 612) se fait vers un dispositif microfluidique.

4. Système de culture cellulaire, **caractérisé en ce qu'**il comprend :
- au moins une chambre de pressurisation (611, 612) comprenant un volume de milieu de culture cellulaire liquide (68), ladite chambre de pressurisation (611, 612) comprenant en outre au moins une entrée de gaz (62) et au moins une sortie de fluide (63) reliant ledit volume de milieu de culture cellulaire liquide (68) et l'extérieur de ladite au moins une chambre de pressurisation (611, 612); et
- au moins un contrôleur de débit gazeux (61) configuré pour injecter par ladite entrée de gaz (62) dans ledit volume de milieu de culture cellulaire liquide (68) un volume contrôlé d'un gaz ou mélange de gaz à l'intérieur de ladite au moins une chambre de pressurisation (611, 612) ; et
**en ce qu'**il met en oeuvre le procédé selon l'une quelconque des revendications 1 à 3.

5. Système de culture cellulaire selon la revendication 4, **caractérisé en ce que** ledit système comprend plusieurs chambres de pressurisation (611, 612) montées en série ou en parallèle les unes des autres et dans lesquelles le fluide est déplacé successivement d'une chambre à l'autre.

6. Système de culture cellulaire selon l'une des revendications 4 et 5, **caractérisé en ce que** ladite au moins une chambre de pressurisation (611, 612) est formée par un réservoir individuel isolé.

7. Système de culture cellulaire selon l'une des revendications 4 et 5, **caractérisé en ce qu'il** comprend en outre :
- une plaque de culture cellulaire multipuits (7) ;
- un dispositif (100, 200) destiné à être adapté sur ladite plaque de culture cellulaire multipuits (7), ledit dispositif (100, 200) comprenant au moins un circuit microfluidique (A) comprenant au moins un canal microfluidique formé dans au moins une plaque microfluidique intégrée (2), ledit circuit microfluidique (A), s'étendant depuis au moins un orifice de raccordement (6) ou au moins un conduit de raccordement fluidique vers un collecteur (5), ledit collecteur (5) comprenant en outre au moins une cavité (52), ladite au moins une cavité (52) formant avec au moins un puits de ladite plaque de culture cellulaire multipuits (7) ladite au moins une chambre de pressurisation (611, 612) ; et
**en ce que** ledit collecteur (5) comprend au moins un ajutage ou au moins un orifice ou au moins un conduit (51), ledit au moins un ajutage ou au moins un orifice ou au moins un conduit (51) prolongeant ledit au moins un canal microfluidique, et formant ledit au moins un circuit microfluidique (A) complet lorsque ledit dispositif (100, 200) est combiné à ladite plaque de culture cellulaire multipuits (7).

8. Système de culture cellulaire selon la revendication 7, **caractérisé en ce que** ledit dispositif (100, 200) comprend au moins un autre circuit microfluidique parallèle intégré (B), indépendant dudit au moins un circuit microfluidique (A), ledit au moins un autre circuit microfluidique parallèle intégré (B) présentant au moins un canal microfluidique formé dans au moins une autre plaque microfluidique parallèle (8) indépendante de ladite plaque microfluidique (2).

9. Système de culture cellulaire selon l'une des revendications 7 et 8, **caractérisé en ce qu'**un fluide injecté s'écoule depuis ledit au moins un orifice de raccordement (6) ou ledit au moins un conduit de raccordement fluidique à l'intérieur dudit au moins un circuit microfluidique (A) vers ledit collecteur (5) et vers ladite plaque de culture cellulaire multipuits (7).

10. Système de culture cellulaire selon l'une des revendications 8 et 9, **caractérisé en ce qu'**un liquide de thermalisation injecté s'écoule depuis ledit au moins un orifice de raccordement (6) ou ledit au moins un conduit de raccordement fluidique à l'intérieur dudit au moins un circuit microfluidique parallèle intégré (B).

## Patentansprüche

1. Verfahren zur Verdrängung eines Fluids und gleichzeitigen Anreicherung eines flüssigen Zellkulturmediums mit Gas, **dadurch gekennzeichnet, dass** es umfasst:
- Injizieren eines kontrollierten Volumens eines Gases oder Gasgemisches mittels mindestens eines Gasdurchflussreglers (61) in das Innere mindestens einer Druckkammer (611, 612), wobei die Injektion durch mindestens einen Gaseinlass (62) in ein Flüssigkeitsvolumen (68) erfolgt, wobei die Injektion ein Sprudeln und ein Rühren des Volumens flüssigen Zellkulturmediums (68) bewirkt;
- Ansammeln des Gases oder Gasgemisches durch Auftrieb in einem hermetisch dichten Raum, der durch das Volumen flüssigen Zellkulturmediums (68) und die mindestens eine Druckkammer (611, 612) gebildet wird, und
- Erhöhen eines Drucks in der mindestens einen Druckkammer (611, 612), bis ein ausreichender kontrollierter Druck von kleiner oder gleich 10 bar erreicht wird, wobei die Erhöhung eine Verdrängung des Volumens flüssigen Zellkulturmediums (68) durch mindestens einen Fluidauslass (63) bewirkt, der das Volumen flüssigen Zellkulturmediums (68) und die Außenseite der mindestens einen Druckkammer (611, 612) verbindet.

2. Verfahren zur Verdrängung eines Fluids und gleichzeitigen Anreicherung eines flüssigen Zellkulturmediums mit Gas nach Anspruch 1, **dadurch gekennzeichnet, dass** das mindestens eine injizierte Gas ausgewählt ist aus O₂, CO₂, N₂ oder deren Gemischen.

3. Verfahren zur Verdrängung eines Fluids und gleichzeitigen Anreicherung eines flüssigen Zellkulturmediums mit Gas nach einem der Ansprüche 1 bis 2, **dadurch gekennzeichnet, dass** die Verdrängung des Volumens flüssigen Zellkulturmediums (68) durch den mindestens einen Fluidauslass (63), der das Volumen flüssigen Zellkulturmediums (68) und die Außenseite der mindestens einen Druckkammer (611, 612) verbindet, zu einer mikrofluidischen Vorrichtung erfolgt.

4. Zellkultursystem, **dadurch gekennzeichnet, dass** es umfasst:
- mindestens eine Druckkammer (611, 612), die ein Volumen flüssigen Zellkulturmediums (68) umfasst, wobei die Druckkammer (611, 612) weiter mindestens einen Gaseinlass (62) und mindestens einen Fluidauslass (63) umfasst, die das Volumen flüssigen Zellkulturmediums (68) und die Außenseite der mindestens einen Druckkammer (611, 612) verbinden; und
- mindestens einen Gasdurchflussregler (61), der so konfiguriert ist, dass er durch den Gaseinlass (62) in das Volumen flüssigen Zellkulturmediums (68) ein kontrolliertes Volumen eines Gases oder Gasgemisches in das Innere der mindestens einen Druckkammer (611, 612) injiziert; und
dadurch, dass es das Verfahren nach einem der Ansprüche 1 bis 3 implementiert.

5. Zellkultursystem nach Anspruch 4, **dadurch gekennzeichnet, dass** das System mehrere Druckkammern (611, 612) umfasst, die in Reihe oder parallel zueinander angebracht sind und in denen das Fluid nacheinander von einer Kammer zur anderen verdrängt wird.

6. Zellkultursystem nach einem der Ansprüche 4 und 5, **dadurch gekennzeichnet, dass** die mindestens eine Druckkammer (611, 612) durch ein isoliertes Einzelreservoir gebildet wird.

7. Zellkultursystem nach einem der Ansprüche 4 und 5, **dadurch gekennzeichnet, dass** es weiter umfasst:
- eine Multiwell-Zellkulturplatte (7);
- eine Vorrichtung (100, 200), die dazu bestimmt ist, auf die Multiwell-Zellkulturplatte (7) angepasst zu werden, wobei die Vorrichtung (100, 200) mindestens einen mikrofluidischen Kreis (A) umfasst, der mindestens einen mikrofluidischen Kanal umfasst, der in mindestens einer integrierten mikrofluidischen Platte (2) gebildet ist, wobei sich der mikrofluidische Kreis (A) von mindestens einer Anschlussöffnung (6) oder mindestens einer fluidischen Anschlussleitung zu einem Sammler (5) erstreckt, wobei der Sammler (5) weiter mindestens einen Hohlraum (52) umfasst, wobei der mindestens eine Hohlraum (52) zusammen mit mindestens einem Well der Multiwell-Zellkulturplatte (7) die mindestens eine Druckkammer (611, 612) bildet; und
dadurch, dass der Sammler (5) mindestens eine Düse oder mindestens eine Öffnung oder mindestens eine Leitung (51) umfasst, wobei die mindestens eine Düse oder mindestens eine Öffnung oder mindestens eine Leitung (51) den mindestens einen mikrofluidischen Kanal verlängert und den mindestens einen vollständigen mikrofluidischen Kreis (A) bildet, wenn die Vorrichtung (100, 200) mit der Multiwell-Zellkulturplatte (7) kombiniert wird.

8. Zellkultursystem nach Anspruch 7, **dadurch gekennzeichnet, dass** die Vorrichtung (100, 200) mindestens einen weiteren integrierten parallelen mikrofluidischen Kreis (B) umfasst, der von dem mindestens einen mikrofluidischen Kreis (A) unabhängig ist, wobei der mindestens eine weitere integrierte parallele mikrofluidische Kreis (B) mindestens einen mikrofluidischen Kanal aufweist, der in mindestens einer weiteren parallelen mikrofluidischen Platte (8) gebildet ist, die von der mikrofluidischen Platte (2) unabhängig ist.

9. Zellkultursystem nach einem der Ansprüche 7 und 8, **dadurch gekennzeichnet, dass** ein injiziertes Fluid von der mindestens einen Anschlussöffnung (6) oder der mindestens einen fluidischen Anschlussleitung in das Innere des mindestens einen mikrofluidischen Kreises (A) zum Sammler (5) und zur Multiwell-Zellkulturplatte (7) strömt.

10. Zellkultursystem nach einem der Ansprüche 8 und 9, **dadurch gekennzeichnet, dass** eine injizierte Thermalisierungsflüssigkeit von der mindestens einen Anschlussöffnung (6) oder der mindestens einen fluidischen Anschlussleitung in das Innere des mindestens einen integrierten parallelen mikrofluidischen Kreises (B) strömt.

## Claims

1. A method for moving a fluid and simultaneously enriching a liquid cell culture medium with gas, **characterised in that** it comprises:
- injecting a controlled volume of a gas or mixture of gases inside at least one pressurisation chamber (611, 612) by means of at least one gas flow controller (61), said injection taking place through at least one gas inlet (62) into a volume of liquid (68), said injection producing bubbling and agitation of said volume of liquid cell culture medium (68);
- accumulating said gas or mixture of gases by buoyancy in a hermetic space formed by said volume of liquid cell culture medium (68) and said at least one pressurisation chamber (611, 612), and
- increasing a pressure in said at least one pressurisation chamber (611, 612) until reaching a sufficient controlled pressure less than or equal to 10 bar, said increase producing a movement of said volume of liquid cell culture medium (68) through at least one fluid outlet (63) connecting said volume of liquid cell culture medium (68) and the exterior of said at least one pressurisation chamber (611, 612).

2. The method for moving a fluid and simultaneously enriching a liquid cell culture medium with gas according to claim 1, **characterised in that** said at least one injected gas is selected from O₂, CO₂, N₂ or mixtures thereof.

3. The method for moving a fluid and simultaneously enriching a liquid cell culture medium with gas according to any one of claims 1 to 2, **characterised in that** said movement of said volume of liquid cell culture medium (68) through said at least one fluid outlet (63) connecting said volume of liquid cell culture medium (68) and the exterior of said at least one pressurisation chamber (611, 612) is towards a microfluidic device.

4. A cell culture system, **characterised in that** it comprises:
- at least one pressurisation chamber (611, 612) comprising a volume of liquid cell culture medium (68), said pressurisation chamber (611, 612) further comprising at least one gas inlet (62) and at least one fluid outlet (63) connecting said volume of liquid cell culture medium (68) and the exterior of said at least one pressurisation chamber (611, 612); and
- at least one gas flow controller (61) configured to inject through said gas inlet (62) into said volume of liquid cell culture medium (68) a controlled volume of a gas or gas mixture inside said at least one pressurisation chamber (611, 612); and
**in that** it implements the method according to any one of claims 1 to 3.

5. The cell culture system according to claim 4, **characterised in that** said system comprises several pressurisation chambers (611, 612) mounted in series or in parallel with each other and wherein the fluid is moved successively from a chamber to the other.

6. The cell culture system according to one of claims 4 and 5, **characterised in that** said at least one pressurisation chamber (611, 612) is formed by an isolated individual reservoir.

7. The cell culture system according to one of claims 4 and 5, **characterised in that** it further comprises:
- a multiwell cell culture plate (7);
- a device (100, 200) intended to be adapted on said multiwell cell culture plate (7), said device (100, 200) comprising at least one microfluidic circuit (A) comprising at least one microfluidic channel formed in at least one integrated microfluidic plate (2), said microfluidic circuit (A), extending from at least one connection orifice (6) or at least one fluidic connection conduit towards a collector (5), said collector (5) further comprising at least one cavity (52), said at least one cavity (52) forming with at least one well of said multiwell cell culture plate (7) said at least one pressurisation chamber (611, 612); and
**in that** said collector (5) comprises at least one nozzle or at least one orifice or at least one conduit (51), said at least one nozzle or at least one orifice or at least one conduit (51) extending said at least one microfluidic channel, and forming said at least one complete microfluidic circuit (A) when said device (100, 200) is combined with said multiwell cell culture plate (7) .

8. The cell culture system according to claim 7, **characterised in that** said device (100, 200) comprises at least one other integrated parallel microfluidic circuit (B), independent of said at least one microfluidic circuit (A), said at least one other integrated parallel microfluidic circuit (B) having at least one microfluidic channel formed in at least one other parallel microfluidic plate (8) independent of said microfluidic plate (2).

9. The cell culture system according to one of claims 7 and 8, **characterised in that** an injected fluid flows from said at least one connection orifice (6) or said at least one fluid connection conduit to the interior of said at least one microfluidic circuit (A) towards said collector (5) and towards said multiwell cell culture plate (7).

10. The cell culture system according to one of claims 8 and 9, **characterised in that** an injected thermalisation liquid flows from said at least one connection orifice (6) or said at least one fluid connection conduit to the interior of said at least one integrated parallel microfluidic circuit (B).
